# EUROPÄISCHE PATENTSCHRIFT

(11) **EP 0 915 075 B1**
(45) Veröffentlichungstag und Bekanntmachung des Hinweises auf die Patenterteilung: **02.07.2003**
(21) Anmeldenummer: 98120070.2
(22) Anmeldetag: 23.10.1998
(51) Int. Cl.: C07C 43/13, C07C 41/09, C07C 29/10, C07C 31/18

(54) **Verfahren zur intermolekularen Veretherung und zur Etherspaltung**
Process for intermolecular etherification and ether cleavage
Procédé d'étherification intermoleculaire et de clivage d'éthers

(30) Priorität: 07.11.1997 DE 19749201
(43) Veröffentlichungstag der Anmeldung: 12.05.1999
(73) Patentinhaber: Perstorp Specialty Chemicals AB, 284 80 Perstorp (SE)
(72) Erfinder: Morawietz, Marcus Dr., 63457 Hanau (DE); Haas, Thomas Dr., 60316 Frankfurt (DE); Burkhardt, Olaf Dr., 63577 Alzenau (DE); Vanheertum, Rudolf Dr., 63796 Kahl (DE)
(74) Vertreter: HOFFMANN - EITLE

(56) Entgegenhaltungen:
- EP-A- 0 462 283
- DE-A- 4 325 753
- DE-A- 4 422 051
- DE-A- 19 510 438
- DATABASE WPI Section Ch, Week 8947 Derwent Publications Ltd., London, GB; Class A41, AN 89-343233 XP002093255 & JP 01 254636 A (DAICEL CHEM IND) , 11. Oktober 1989
- DATABASE WPI Section Ch, Week 9237 Derwent Publications Ltd., London, GB; Class A41, AN 92-303557 XP002093256 & JP 04 208242 A (NIPPON SYNTHETIC CHEM IND)
- C. MONTASSIER: "Polyol conversion into furanic derivatives on bimetallic catalysts; nature of the catalytic sites" JOURNAL OF MOLECULAR CATALYSIS, Bd. 91, 1994, Seiten 119-128, XP002093254

## Beschreibung

Die Erfindung betrifft ein Verfahren zur intermolekularen Veretherung von ein- oder mehrwertigen Alkoholen, umfassend Behandlung des Alkohols in wäßriger Phase in Gegenwart eines sauren Katalysators bei einer Temperatur von mindestens 100 °C. Die Erfindung richtet sich insbesondere auf die intermolekulare Veretherung von Diolen, Triolen und Tetrolen unter Bildung der korrespondierenden Di-, Tetra- und Hexahydroxyether.

Bei der intermolekularen Veretherung von Alkoholen in Gegenwart von Wasser und einem sauren Katalysator stellt sich in bekannter Weise ein Gleichgewicht mit dem gebildeten Ether ein. Demgemäß lassen sich auch beim erfindungsgemäßen Verfahren unter gleichen Reaktionsbedingungen Ether bilden und spalten. Veretherungsprodukte von mehrwertigen Alkoholen, nachfolgend allgemein als Polyhydroxyether bezeichnet, sind Rohstoffe für unterschiedlichste Einsatzgebiete, darunter zur Herstellung von Polyesterharzen, Schmiermitteln, PVC-Stabilisatoren und Weichmachern.

Obgleich es sich bei der intermolekularen Veretherung um eine Grundreaktion der organischen Chemie handelt, ergeben sich bei der Veretherung von Diolen, Triolen, Tetrolen und anderen Polyolen oft größere Probleme durch eine ungenügende Selektivität an dem gewünschten Hydroxyether sowie durch die Bildung von Polymeren sowie färbenden Verunreinigungen. Bei der Veretherung von 1,2-Diolen läßt sich das Problem durch die Verwendung von Epoxiden beheben. Bei der Veretherung von Diolen mit mehr als 2 C-Atomen zwischen den Hydroxylgruppen, wie 1,3-Propandiol oder 1,4-Butandiol, zwecks Gewinnung von Monoveretherungsprodukten wird meistens eine Hydroxylfunktion geschützt, bevor die Veretherung durchgeführt wird - siehe J. Chem. Soc. Perkin Trans 1(1), (1992), 153-156. Damit wird das Veretherungsverfahren aber aufwendig und teuer.

Die an technischer Bedeutung gewinnenden Polyhydroxyether vom Typ des Di- und Tripentaerythritols (Di- und Tripenta) sowie der entsprechenden Polyhydroxyether von Trimethyolethan (TME) und Trimethylolpropan (TMP) lassen sich als Koppelprodukte bei der bekannten Herstellung von Penta, TME und TMP über eine Aldol- und Cannizzaro-Reaktion durch Variation der Reaktionsparameter gewinnen. Die Ausbeuten an den Ethern sind meist begrenzt und stets an die Herstellung des Hauptproduktes Penta, TME oder TMP gekoppelt - US-Patent 2,441,597 und JP-A 8-176048.

Gemäß JP-A 4-208242 läßt sich Pentraerythritol (Penta) in der Schmelze in Gegenwart von Schwefelsäure mit einer Dipenta-Selektivität von 35 %, bezogen auf einen Penta-Umsatz von 10 bis 15 % verethern. Ein ähnliches Verfahren zur Herstellung von Dipenta aus Penta, wobei jedoch als saurer Katalysator ein Phosphat von Ti, Al, Cr und Zr eingesetzt wird, ist aus der EP-B 0 462 283 bekannt.

Nachteile der in Abwesenheit eines Lösungsmittels betriebenen Verfahrens sind der meist hohe Anteil an höheren Veretherungsprodukten, wie Tri-, Tetra- und Polypenta sowie die Bildung stark gefärbter Verunreinigungen. Nachteilig ist wiederum die Verwendung eines organischen Lösungsmittels. Da der Umsatz an Penta zwecks Erhalt einer hohen Dipenta-Selektivität niedrig gehalten werden muß, vergrößert sich zudem der Aufwand für die Aufarbeitung. Das Verfahren des genannten EP-Patents kann auch in Gegenwart von Wasser oder einem aprotischen dipolaren Lösungsmittel durchgeführt werden: Die Selektivität bezüglich Dipenta kann bei Verwendung von Sulfolan als Lösungsmittel etwa 70 % erreichen, der Umsatz an Penta 15 %; nachteilig ist aber das Erfordernis, ein organisches Lösungsmittel einsetzen zu müssen.

Durch Behandeln von Penta mit Harnstoff (8 h, 190 °C) bilden sich etwa 8 % Dipenta und 3 % Tripenta (JP-A 7-76541). Bei zusätzlicher Verwendung eines Katalysators auf der Basis von Zirkonium, Titan oder Zinn sowie eines dipolaren aprotischen Lösungsmittels, wie Sulfolan, kann die Dipenta-Ausbeute auf 20 bis 25 % erhöht werden (JP-A 7-258139, JP-A 7-188086 und JP-A 7-165653). Hinzu kommt, daß das Reaktionsgemisch der Veretherung den Katalysator in teilweise gelöster Form enthält; damit wird die Abtrennung erschwert.

Alternative Verfahren basieren auf der teilweisen Veresterung der Hydroxy-Funktionen von Polyolen, um in einer nachgeschalteten Veretherung die Entstehung von Oligomeren beziehungsweise Polymeren zu unterdrücken (EP-B 0 550 611). Der Nachteil dieser Reaktionen sind die mäßigen Gesamtausbeuten sowie die vorgeschaltete Veresterung und die anschließend notwendige Esterspaltung, um den Polyhydroxyether als Reinprodukt zu erhalten. Durch die Esterspaltung fallen große Mengen der entsprechenden Carbonsäure-Salze an, die entsorgt werden müssen.

Während die intramolekulare Veretherung von Zuckeralkoholen unter hydrierenden Bedingungen mit H₂ in Gegenwart eines Hydrierkatalysators bekannt ist (J. Mol. Cat. 91 (1994) 119-128), wurden diese Bedingungen nie auf die intermolekulare Veretherung angewandt. Zudem erwies sich die Katalysatorstandzeit als begrenzt und die Selektivität als mäßig.

Aufgabe der vorliegenden Erfindung war demgemäß, ein verbessertes Verfahren aufzuzeigen, das es gestattet, Dialkylether, Di- und Polyhydroxyether durch intermolekulare Veretherung aus den entsprechenden monomeren Alkoholen in guter Ausbeute, insbesondere aber hoher Selektivität bezüglich der Monoether zu gewinnen und hierbei die Bildung von höheren Oligomeren, Polymeren, ungesättigten Nebenprodukten und deren polymeren Folgeprodukten sowie Verfärbungen weitgehend zu unterdrücken.

Die Aufgabe wird gelöst durch ein Verfahren zur intermolekularen Veretherung von ein- oder mehrwertigen Alkoholen, umfassend Behandlung des Alkohols in wäßriger Phase in Gegenwart eines sauren Katalysators bei einer Temperatur von mindestens 100 °C, das dadurch gekennzeichnet ist, daß man die Behandlung in Gegenwart eines säurestabilen Hydrierkatalysators unter Wasserstoffatmosphäre durchführt.

Es wurde überraschend gefunden, daß durch Verwendung einer Kombination aus einem sauren Katalysator und einem Hydrierkatalysator, und Verwendung von Wasser als Lösungsmittel bei der Veretherung eines mehrwertigen Alkohols unter H₂-Atmosphäre eine hohe Selektivität an Monoethern erhältlich ist. Zudem werden praktisch keine höheren oligomeren und polymeren Verbindungen gebildet und das Reaktionsgemisch bleibt im wesentlichen farblos. Aus Dihydroxyalkanen lassen sich so Bis(hydroxyalkyl)ether, aus Trihydroxyalkanen Bis(dihydroxyalkyl)ether und aus Tetrahydroxyalkanen Bis (trihydroxyalkyl)ether gewinnen.

Dem erfindungsgemäßen Verfahren zur Veretherung sind primäre oder sekundäre Alkohole, vorzugsweise primäre Alkohole, mit einer oder mehreren Hydroxylgruppen, zugänglich. Wesentlich ist, daß der Alkohol unter den Reaktionsbedingungen eine ausreichende Löslichkeit in Wasser oder wäßrigen Lösungen aufweist. Die Alkohole können weitere funktionelle Gruppen enthalten, soweit diese hydrolysestabil sind und unter den erfindungsgemäßen Hydrierbedingungen nicht hydriert werden. Besonders bevorzugt werden aliphatische Diole, Triole und Tetrole der Veretherung zugeführt. Beispiele für Diole sind Propan-1,3-diol, Butan-1,4-diol, Hexan-1,6-diol, Neopentylglykol; Beispiele für Triole sind Glycerin, Trimethylolethan (TME) Trimethlolpropan (TMP), Hexan-1,2,6-triol; ein Beispiel für Tetrole ist Pentaerythrit.

Die erfindungsgemäße Veretherung erfolgt in Gegenwart von Wasser. Bei Bedarf kann die zu verethernde Lösung außerdem ein- oder mehrwertigen Alkohol und Wasser zusätzlich andere säure- und hydrierstabile Lösungsmittel, wie ein aprotisches dipolares Lösungsmittel, enthalten; bevorzugt wird aber der Einsatz einer rein wäßrigen Lösung. Zweckmäßigerweise werden der Alkohol und das Wasser im Gewichtsverhältnis im Bereich von 10 zu 1 bis 1 zu 5, vorzugsweise im Bereich von 4 zu 1 bis 1 zu 4 und insbesondere 3 zu 2 bis 1 zu 2 eingesetzt.

Die Umsetzung erfolgt bei einer Temperatur von mindestens 100 °C, üblicherweise bei einer Temperatur im Bereich von 120 bis 380 °C. Bevorzugt wird eine Temperatur im Bereich von 120 bis 300 °C, besonders bevorzugt 180 bis 280 °C.

Zur Etherbildung ist die Anwesenheit eines sauren Katalysators erforderlich. Bezüglich der Hammet'schen Säuredefinition wird verwiesen auf: Studies in surface science and catalysis, Vol. 51 (1989): New solid acid and basis" by Tanabe et al., Seite 5. Einsetzbar sind Mineralsäuren, wie H₂SO₄, HCl und H₃PO₄, organische Carbönund Sulfonsäuren sowie saure Feststoffkatalysatoren, deren Hₒ-Wert der Hammet'schen Säurefunktion kleiner -2, insbesondere kleiner -3 ist. Mineralsäuren werden weniger bevorzugt, weil diese nach der Umsetzung neutralisiert und die Salze aus dem Reaktionsgemisch abgetrennt und entsorgt werden müssen.

Um die Aufarbeitung des Reaktionsgemischs der Etherbildung möglichst einfach zu gestalten, wird gemäß einer bevorzugten Ausführungsform eine Carbonsäure, deren Siedepunkt unterhalb desjenigen des herzustellenden Ethers beziehungsweise Alkohols liegt, insbesondere eine C₁- bis C₁₂-Monocarbonsäure, als Katalysator eingesetzt. Besonders bevorzugt wird eine Carbonsäure aus der Reihe Ameisensäure, Essigsäure und Propionsäure. Derartige Carbonsäuren lassen sich destillativ aus dem Reaktionsgemisch abtrennen und dann recyclieren.

Bei den sauren Feststoffkatalysatoren mit Hₒ kleiner +2 handelt es sich um Stoffe aus den Reihen: natürliche und synthetische silikatische Stoffe, wie Montmorillonit, Mordenit und saure Zeolithe; an anorganischen Trägerstoffen, wie SiO₂, Al₂O₃ oder TiO₂, fest gebundene Säuren, wie insbesondere Phosphoroxide/-säuren; Oxide, wie gamma-Al₂O₃, TiO₂, ZrO₂, SnO₂, Bi₂O₅, Sb₂O₅, MoO₃, WO₃; Mischoxide, wie SiO₂-Al₂O₃, SiO₂-TiO₂, Al₂O₃-ZnO, SiO₂-ZrO₂, SiO₂-SnO₂, SiO₂-MoO₃, SiO₂-WO₃; Heteropolysäuren, zum Beispiel Polywolframatosilikate und Polywolframatophosphate; Metallsalze, wie AlPO₄, FePO₄, Zn₃(PO₄)₂, Mg₃(PO₄)₂, Ti₃(PO₄)₄, Zr₃(PO₄)₄; Kationenaustauscher, wie Sulfonatgruppen enthaltende Austauscher auf der Basis von Polystyrol, polymeren perfluorierten Harzen oder vorzugsweise Organopolysiloxanen (Deloxan® der Degussa AG). Besonders bevorzugte saure Feststoffkatalysatoren für die erfindungsgemäße Etherbildung sind Zeolithe vom Typ H-Y, H-Beta und H-ZSM 5.

Die Einsatzmenge von im.Reaktionsgemisch löslichen sauren Katalysatoren liegt im allgemeinen im Bereich von 0,1 bis 20 Gew.-%, insbesondere 0,5 und 10 Gew.-%, jeweils bezogen auf den umzusetzenden ein- oder mehrwertigen Alkohol. Die Einsatzmenge fester saurer Katalysatoren richtet sich sowohl nach deren Aktivität und der gewählten Reaktionstemperatur; die Einsatzmenge läßt sich durch orientierende Versuche einfach ermitteln.

Erfindungswesentliches Merkmal ist, daß zusätzlich zum sauren Katalysator ein üblicher Hydrierkatalysator anwesend ist und die Etherbildung in einer Wasserstoffatmosphäre durchgeführt wird. Der Wasserstoffpartialdruck liegt im allgemeinen im Bereich von mindestens 0,1 bis 15 MPa, vorzugsweise im Bereich von 1 bis 15 MPa und insbesondere 3 bis 10 MPa.

Als Hydrierkatalysatoren können zwar homogene und heterogene Katalysatoren eingesetzt werden, jedoch werden heterogene Katalysatoren bevorzugt, weil damit eine einfache Abtrennung des Katalysators vom Reaktionsgemisch, etwa durch Filtration, möglich ist. Übliche Hydrierkatalysatoren enthalten als wirksame Komponente ein Edelmetall aus der Reihe Ru, Rh, Pd und Pt, oder ein Übergangsmetall aus der Reihe Cu, Cr, Co, Ni, Fe, darunter insbesondere Raney-Katalysatoren und Chromit-Katalysatoren; einsetzbar sind auch Bimetall-Katalysatoren aus einem Übergangsmetall und Edelmetall. Die Verwendung eines ein oder mehrere Übergangsmetalle enthaltenden Hydrierkatalysators ist nur dann zweckmäßig, wenn der Katalysator unter den Reaktionsbedingungen eine ausreichende Säurestabilität aufweist.

Bevorzugte Hydrierkatalysatoren für das erfindungsgemäße Verfahren sind Edelmetallkatalysatoren in metallischer Form, wie sogenannte Mohre von Ru, Rh und insbesondere Pd und Pt, oder in an einen Träger gebundener Form. Geeignete Trägermaterialien für Ru, Rh, Pd und Pt sind Aktivkohle, Aluminiumoxid, SiO₂, TiO₂ und andere Metalloxide sowie Silikate. Die Edelmetallmenge trägergebundener Edelmetallkatalysatoren liegt meist im Bereich von 0,0001 bis 10 Gew.-%, bei Pd vorzugsweise im Bereich von 0,01 bis 1 Gew.-%, bei Ru vorzugsweise im Bereich von 0,01 bis 0,1 Gew.-%. Die optimale Einsatzmenge an Edelmetallkatalysatoren, welche von der Aktivität des Katalysators, der Reaktionstemperatur und dem H₂-Druck abhängt, wird der Fachmann durch orientierende Versuche ermitteln. Im allgemeinen liegt die Einsatzmenge bei handelsüblichen Trägerkatalysatoren im Bereich von 0,001 bis 10 Gew.-%, insbesondere 0,01 bis 1 Gew.-%, bezogen auf den zu verethernden Alkohol. Edelmetallkatalysatoren in Form eines Mohrs oder trägergebunden lassen sich leicht recyclieren, sie weisen eine höhere Standzeit auf als Bimetall-Katalysatoren auf der Basis eines Edelmetalls und eines Übergangsmetalls, wie sie bei der vorbekannten intramolekularen Veretherung (Cyclodehydratation) in Abwesenheit eines sauren Katalysators eingesetzt wurden.

Das Verfahren kann diskontinuierlich oder kontinuierlich betrieben werden. Bei der Veretherung können der ein- oder mehrwertige Alkohol und Wasser vor dem Reaktor gemischt oder parallel dem Reaktor zugeführt werden. Bei Verwendung einer im Reaktionsgemisch löslichen Säure als Katalysator wird dies dem Reaktionspartner, dem Wasser oder Gemisch aus beiden zugesetzt oder separat in den Reaktor eingeführt. Der feste Hydrierkatalysator kann als Suspensionskatalysator oder als Festbett eingesetzt werden. Bei Verwendung eines festen sauren Katalysators kann dieser analog zum Hydrierkatalysator als Suspension oder als Festbett zur Anwendung gelangen. Es ist auch möglich, einen sowohl saure als auch hydrierwirksame Funktionen enthaltenden Katalysator, beispielsweise einen teilweise mit einem Edelmetall beladenen Zeolithen, einzusetzen. Die optimale Reaktionszeit kann der Fachmann durch orientierende Versuche leicht ermitteln.

Das Reaktionsgemisch läßt sich nach beendeter Umsetzung beziehungsweise Gleichgewichtseinstellung in einfacher Weise aufarbeiten. Diese Aufarbeitung kann die Filtration eines festen sauren Katalysators und eines heterogenen Hydrierkatalysators umfassen. Bei Verwendung eines destillierbaren sauren Katalysators, wie einer bevorzugten niederen Carbonsäure, und eines heterogenen Hydrierkatalysators umfaßt die Aufarbeitung die Filtration des Hydrierkatalysators und destillative Abtrennung des sauren Katalysators und des Wassers. Das verbleibende Reaktionsgemisch wird destillativ und/oder extraktiv und/oder durch Kristallisation, vorzugsweise durch Destillation und/oder Kristallisation, aufgearbeitet. Die Analyse der Zusammensetzung des Reaktionsgemischs oder einzelner Fraktionen erfolgt über GC- oder HPLC-Chromatographie. Polyole und Polyolether werden vorzugsweise vor der Analytik mit Hexamethyldisilazan /DMF silyliert und anschließend mit GC-Chromatographie analysiert.

Vorteile des erfindungsgemäßen Verfahrens zur Veretherung sind die gegenüber vorbekannten Verfahren hohe Selektivität an Monoethern, die weitgehende Abwesenheit von polymeren Etheralkoholen und farbgebenden Nebenprodukten. Ein weiterer Vorteil ist die Einfachheit des Verfahrens, da vor und nach der Veretherung keine zusätzlichen Verfahrensstufen nötig sind und kein Koppelprodukt entsteht. Bei einer der bevorzugten Ausführungsformen - Einsatz von Carbonsäuren oder sauren Feststoffen als Katalysator - müssen ferner keine Salze entfernt und entsorgt werden; zudem sind die sauren Katalysatoren ebenso recyclierbar wie die Hydrierkatalysatoren.

### Beispiel 1: Veretherung von 1,3-Propandiol

Es werden 1750 g 1,3-Propandiol, 1750 g Wasser, 17,5 g 3 % Pd/C und 175 g Propionsäure in einem 5 l-Autoklav gegeben. Bei Raumtemperatur werden 60 bar Wasserstoff aufgepreßt und anschließend wird das Reaktionsgemisch auf 250 °C erwärmt. Bei dieser Temperatur rührt das Gemisch eine Stunde. Nach dem Abkühlen wird der Autoklav bei 80 °C entleert, der Katalysator abfiltriert und eine repräsentative Probe entnommen. Das Wasser wird am Rotationsverdampfer vollständig entfernt, es werden 1630 g einer farblosen Flüssigkeit erhalten und der Rückstand wird mittels HPLC quantitativ analysiert. Die Ergebnisse sind in der Tabelle 1 zusammengefaßt.

### Beispiel 2: Veretherung von Trimethylolpropan

Die Reaktion wird analog der im Beispiel 1 beschriebenen Vorschrift durchgeführt, allerdings wird anstelle von 1750 g 1,3-Propandiol 1750 g Trimethylolpropan eingesetzt. Die Ausbeute beträgt 1713 g einer farblosen Flüssigkeit. Die Ergebnisse sind in der Tabelle 1 zusammengefaßt.

### Beispiel 3: Veretherung von Glycerin

Die Reaktion wird analog der im Beispiel 1 beschriebenen Vorschrift durchgeführt, allerdings wird anstelle von 1750 g 1,3-Propandiol 1750 g Glycerin eingesetzt. Die Ausbeute beträgt 1688 g einer farblosen Flüssigkeit. Es werden drei verschiedene Di-Glycerin-Isomere erhalten. Die Ergebnisse sind in der Tabelle 1 zusammengefaßt.

### Beispiel 4: Veretherung von Pentaerythritol

Es werden 1750 g Pentaerythritol, 1750 g Wasser, 17,5 g 3 % Pd/C und 175 g Propionsäure in einem 5 l-Autoklav gegeben. Bei Raumtemperatur werden 60 bar Wasserstoff aufgepreßt und anschließend wird das Reaktionsgemisch auf 250 °C erwärmt. Bei dieser Temperatur rührt das Gemisch eine Stunde. Nach dem Abkühlen wird der Autoklav bei 80 °C entleert, zum Auflösen des ausgefallenen Pentas erwärmt, der Katalysator abfiltriert und eine repräsentative Probe entnommen. Das Wasser wird am Rotationsverdampfer vollständig entfernt, es werden 1740 g eines farblosen Feststoffes erhalten und der Rückstand wird mittels GC quantitativ analysiert. Die Ergebnisse sind in der Tabelle 1 zusammengefaßt.

### Beispiel 5: Veretherung von Pentaerythritol

Durchführung analog Beispiel 4, nur daß die Reaktion bei 280 °C innerhalb einer Stunde durchgeführt wurde. Es werden 1720 g eines farblosen Feststoffes erhalten. Die Ergebnisse sind in der Tabelle 1 zusammengefaßt.

### Beispiel 6: Veretherung von Pentaerythritol

Durchführung analog Beispiel 4, nur daß die Reaktion bei 290 °C innerhalb einer Stunde durchgeführt wurde. Es werden 1701 g eines farblosen Feststoffes erhalten. Die Ergebnisse sind in der Tabelle 1 zusammengefaßt.

### Beispiel 7: Veretherung von Pentaerythritol

Durchführung analog Beispiel 4, nur daß anstelle der Propionsäure 20 g Beta-Zeolith zugegeben wurde. Die Reaktion wurde bei 250 °C innerhalb einer Stunde durchgeführt wurde. Es werden 1735 g eines farblosen Feststoffes erhalten. Die Ergebnisse sind in der Tabelle 1 zusammengefaßt.

### Beispiel 8: Veretherung von Pentaerythritol

Durchführung analog Beispiel 7, nur daß die Reaktion bei 280 °C innerhalb einer Stunde durchgeführt wurde. Es werden 1681 g eines farblosen Feststoffes erhalten. Die Ergebnisse sind in der Tabelle 1 zusammengefaßt.

### Vergleichsbeispiel 1: Veretherung von Pentaerythritol ohne reduktive Reaktionsbedingungen

Durchführung analog Beispiel 5, nur daß die Reaktion ohne Hydrierkatalysator und unter Stickstoffatmosphäre durchgeführt wird. Es werden 1723 g eines braungefärbten Feststoffes erhalten. Die Ergebnisse sind in der Tabelle 1 zusammengefaßt.

**Tabelle 1**

| **Reaktion** | **Ausbeute** [g] | **Umsatz** [%] | **Ausbeute** [g] | **Selektivität** Produkt [% bez. umges. Polyol] | **Selektivität** polymere Produkte [% bez. umges. Polyol] |
|---|---|---|---|---|---|
| B 1 | 1545 g Propandiol | 11,7 | 45,1 g Di-Propandiol | 25,5 | < 1 |
| B 2 | 1635 g TMP | 6,6 | 53,1 g Di-TMP | 49,5 | < 1 |
| B 3 | 1575 g Glycerin | 10,0 | 52,5 g Di-Glycerin | 33,2 | < 1 |
| B 4 | 1642 g Penta | 6,3 | 55,3 g Dipenta | 54,8 | < 1 |
| B 5 | 1616 g Penta | 7,8 | 64,4 g Dipenta | 51,8 | < 1 |
| B 6 | 1554 g Penta | 11,2 | 78,6 g Dipenta | 42,9 | < 1 |
| B 7 | 1630 g Penta | 6,9 | 61,6 g Dipenta | 54,5 | < 1 |
| B 8 | 1481 g Penta | 15,4 | 104,0 g Dipenta | 41,5 | < 1 |
| VB 1 | 1426 g Penta | 18,8 | 41,1 g Dipenta | 13,3 | 3 |

Bei allen Versuchen beträgt die Selektivität der Veresterungsprodukte maximal 5 bis 10 % der Theorie.

## Patentansprüche

1. Verfahren zur intermolekularen Veretherung von einoder mehrwertigen Alkoholen,
umfassend Behandlung des Alkohols
in wäßriger Phase in Gegenwart eines sauren Katalysators bei einer Temperatur von mindestens 100 °C,
**dadurch gekennzeichnet,**
**daß** man die Behandlung in Gegenwart eines säurestabilen Hydrierkatalysators unter Wasserstoffatmosphäre durchführt.

2. Verfahren nach Anspruch 1,
**dadurch gekennzeichnet,**
**daß** man mehrwertige Alkohole, insbesondere Alkohole aus der Reihe der Diole, Triole und Tetrole, in Gegenwart von Wasser unter Bildung von Hydroxyethern verethert.

3. Verfahren nach Anspruch 1 oder 2,
**dadurch gekennzeichnet,**
**daß** man die Behandlung bei einer Temperatur im Bereich von 120 bis 380 °C unter einem H₂-Partialdruck von 1 bis 15 MPa durchführt.

4. Verfahren nach Anspruch 3,
**dadurch gekennzeichnet,**
**daß** man die Behandlung bei einer Temperatur im Bereich von 180 bis 280 °C und einem H₂-Druck im Bereich von 3 bis 10 MPa durchführt.

5. Verfahren nach einem der Ansprüche 1 bis 4,
**dadurch gekennzeichnet,**
**daß** man als Hydrierkatalysator einen ein oder mehrere Edelmetalle aus der Reihe Ruthenium, Rhodium, Palladium und Platin in elementarer Form oder als Edelmetallverbindung enthaltenden Katalysator einsetzt.

6. Verfahren nach einem der Ansprüche 1 bis 5,
**dadurch gekennzeichnet,**
**daß** man den Hydrierkatalysator in einer Menge von 0,001 bis 10 Gew.-%, insbesondere 0,01 bis 1 Gew.-%, bezogen auf den zu verethernden Alkohol, einsetzt.

7. Verfahren nach einem der Ansprüche 1 bis 6,
**dadurch gekennzeichnet,**
**daß** man als sauren Katalysator eine aliphatische Carbonsäure mit 1 bis 10 C-Atomen, insbesondere eine Monocarbonsäure aus der Reihe Ameisensäure, Essigsäure und Propionsäure, verwendet.

8. Verfahren nach einem der Ansprüche 1 bis 7,
**dadurch gekennzeichnet,**
**daß** man den sauren Katalysator in einer Menge von 0,1 bis 20 Gew.-%, insbesondere 0,5 bis 10 Gew.-%, jeweils bezogen auf das Polyol, einsetzt.

9. Verfahren nach einem der Ansprüche 1 bis 8,
**dadurch gekennzeichnet,**
**daß** man als sauren Katalysator einen heterogenen Katalysator aus der Reihe saurer Zeolithe, saurer Metalloxide, Phosphate und Silikate sowie sulfonsäuregruppenenthaltender Organopolysiloxane einsetzt.

10. Verfahren nach einem der Ansprüche 1 bis 9,
**dadurch gekennzeichnet,**
**daß** man einen saure und hydrierwirksame Funktionen enthaltenden Katalysator auf der Basis eines mit einem Edelmetall aus der Reihe Pd, Pt, Ru und Rh beladenen Zeoliths mit einem Hₒ-Wert von kleiner +2, insbesondere kleiner -3, verwendet.

## Claims

1. Process for intermolecular etherification of monohydric or polyhydric alcohols, comprising treatment of the alcohol in aqueous phase in the presence of an acid catalyst at a temperature of at least 100°C, **characterised in that** the treatment is carried out in the presence of an acid-stable hydrogenation catalyst under hydrogen atmosphere.

2. Process according to claim 1, **characterised in that** polyhydric alcohols, in particular alcohols from the series of diols, triols and tetrols, are etherified in the presence of water with formation of hydroxyethers.

3. Process according to claim 1 or 2, **characterised in that** the treatment is carried out at a temperature in the range from 120 to 380°C under an H₂ partial pressure of 1 to 15 MPa.

4. Process according to claim 3, **characterised in that** the treatment is carried out at a temperature in the range from 180 to 280°C and an H₂ pressure in the range from 3 to 10 MPa.

5. Process according to one of claims 1 to 4, **characterised in that** a catalyst containing one or more noble metals from the series ruthenium, rhodium, palladium and platinum in elemental form or as a noble metal compound is used as hydrogenation catalyst.

6. Process according to one of claims 1 to 5, **characterised in that** the hydrogenation catalyst is used in a quantity of 0.001 to 10 wt.%, in particular 0.01 to 1 wt.%, based on the alcohol to be etherified.

7. Process according to one of claims 1 to 6, **characterised in that** an aliphatic carboxylic acid having 1 to 10 C atoms, in particular a monocarboxylic acid from the series formic acid, acetic acid and propionic acid, is used as acid catalyst.

8. Process according to one of claims 1 to 7, **characterised in that** the acid catalyst is used in a quantity of 0.1 to 20 wt.%, in particular 0.5 to 10 wt.%, in each case based on the polyol.

9. Process according to one of claims 1 to 8, **characterised in that** a heterogeneous catalyst from the series acid zeolites, acid metal oxides, phosphates and silicates as well as organopolysiloxanes containing sulphonic acid groups is used as acid catalyst.

10. Process according to one of claims 1 to 9, **characterised in that** an acid catalyst containing hydrogenation-effective functions based on a zeolite charged with a noble metal from the series Pd, Pt, Ru and Rh and having an Hₒ value of less than +2, in particular less than -3, is used.

## Revendications

1. Procédé d'éthérification intermoléculaire de mono- ou polyalcools, incluant le traitement de l'alcool en phase aqueuse en présence d'un catalyseur acide à une température de 100°C au moins, **caractérisé en ce que** le traitement est réalisé en présence d'un catalyseur d'hydrogénation acido-stable sous atmosphère hydrogénée.

2. Procédé selon la revendication 1 **caractérisé en ce qu'**on éthérifie des polyalcools, en particulier des alcools faisant partie du groupe des diols, des triols et des tétrols, en présence d'eau avec formation d'hydroxyéthers.

3. Procédé selon la revendication 1 ou 2 **caractérisé en ce que** le traitement est conduit à une température dans le domaine de 120 à 380°C et sous une pression partielle d' H₂ de 1 à 15 Mpa.

4. Procédé selon la revendication 3 **caractérisé en ce que** le traitement est conduit à une température dans le domaine de 180 à 280°C et sous une pression partielle d' H₂ de 3 à 10 Mpa.

5. Procédé selon l'une des revendications 1 à 4 **caractérisé en ce qu'**on utilise comme catalyseur d'hydrogénation un ou plusieurs métaux nobles faisant partie du groupe comprenant le ruthénium, le rhodium, le palladium et le platine sous forme élémentaire ou sous forme d'un catalyseur contenant un composé de métal noble.

6. Procédé selon l'une des revendications 1 à 5 **caractérisé en ce qu'**on utilise le catalyseur d'hydrogénation dans une quantité allant de 0,001 à 10% en poids, en particulier allant de 0,01 à 1% sur le poids de l'alcool à éthérifier.

7. Procédé selon l'une des revendications 1 à 6 **caractérisé en ce que** l'on utilise comme catalyseur acide un acide carboxylique aliphatique avec 1 à 10 atomes de carbone, en particulier un acide monocarboxylique faisant partie du groupe comprenant l'acide formique, l'acide acétique et l'acide propionique.

8. Procédé selon l'une des revendications 1 à 7 **caractérisé en ce que** l'on utilise le catalyseur acide dans une quantité allant de 0,1 à 20% en poids, en particulier allant de 0,5 à 10% en poids, sur chaque polyol.

9. Procédé selon l'une des revendications 1 à 8 **caractérisé en ce que** l'on utilise comme catalyseur acide un catalyseur hétérogène faisant partie du groupe comprenant les zéolithes acides, les oxydes de métaux acides, les phosphates et les silicates ainsi que les organopolysiloxanes comprenant un groupe d'acide sulfonique.

10. Procédé selon l'une des revendications 1 à 9 **caractérisé en ce qu'**on utilise un catalyseur contenant des fonctions acides et efficaces pour l'hydrogénation à base d'un zéolithe chargé d'un métal noble faisant partie du groupe comprenant le Pd, le Pt, le Ru et le Rh avec une valeur H₀ plus petite que +2, en particulier plus petite que -3.
